(19) Europäisches Patentamt
European Patent Office
Office européen des brevets

(11) EP 0 557 169 B1

(12) **FASCICULE DE BREVET EUROPEEN**

(45) Date de publication et mention de la délivrance du brevet:
**27.12.1996 Bulletin 1996/52**

(51) Int Cl.[6]: **C07C 51/377**, B01J 23/40, B01J 23/44

(21) Numéro de dépôt: **93400364.1**

(22) Date de dépôt: **12.02.1993**

(54) **Catalyseur de déshalogénation d'acides carboxyliques alphahalogénés, son utilisation pour purifier l'acide monochloracétique**

Katalysator zur Enthalogenierung von Alpha-halogenierten Carbonsäuren und seine Verwendung zur Reinigung von Monochloressigsäure

Catalyst for the dehalogenation of alpha-halogenated carboxylic acids and the use thereof for purifying monochloroacetic acid

(84) Etats contractants désignés:
**DE ES FR GB NL SE**

(30) Priorité: **19.02.1992 FR 9201876**

(43) Date de publication de la demande:
**25.08.1993 Bulletin 1993/34**

(73) Titulaire: **ELF ATOCHEM S.A.**
**92800 Puteaux (FR)**

(72) Inventeur: **Correia, Yves**
**F-01460 Chateau Arnoux (FR)**

(56) Documents cités:
**EP-A- 0 453 690**
**FR-A- 2 196 196**
**US-A- 3 901 660**
**DE-B- 2 323 777**
**FR-A- 2 276 876**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 557 169 B1

Printed by Jouve, 75001 PARIS (FR)

## Description

La présente invention concerne un catalyseur de déshalogénation d'acides carboxyliques alphahalogénés, son utilisation pour purifier l'acide monochloracétique. Elle est particulièrement utile pour éliminer l'acide dichloracétique (ADCA) contenu dans l'acide monochloracétique (AMCA). La synthèse de l'acide monochloracétique à l'échelle industrielle se fait par la chloration de l'acide acétique, mais il se forme inévitablement de l'acide dichloracétique et parfois un peu d'acide trichloracétique. On obtient donc à l'issue de la chloration un mélange constitué d'acide monochloracétique, d'acide dichloracétique, de traces de trichloracétique et d'acide acétique qui n'a pas réagi. Compte-tenu de la proximité des points d'ébullition de l'AMCA (189°C) et de l'ADCA (194°C), il est pratiquement impossible de les séparer par distillation. Par contre, il est très simple d'hydrogéner ce mélange pour convertir l'ADCA en AMCA selon la réaction :

$$CHCl_2COOH + H_2 \rightarrow CH_2ClCOOH + HCl$$

Cette hydrogénation n'est pas totalement sélective et on observe aussi une rétrogradation d'AMCA en acide acétique :

$$CH_2ClCOOH + H_2 \rightarrow CH_3COOH + HCl$$

Cette réaction se fait avec un catalyseur et sous-produit de l'acétaldéhyde qui a l'inconvénient de générer des produits de condensation.

Le brevet FR 1 581 391 décrit un tel procédé en phase liquide, basé sur un catalyseur constitué de silice sous forme de cylindres de longueur 8 mm et 3,5 mm de diamètre, ayant une teneur en palladium de 0,5% en poids.

Le brevet FR 2 046 424 décrit dans son exemple 4 un catalyseur de purification d'AMCA constitué d'une poudre de silice de 0,05 à 0,1 mm chargée de palladium et opérant en lit fluidisé en phase liquide.

Le brevet GB 1 188 745 décrit un catalyseur formé de silice en cylindres de longueur 8mm et de diamètre 3,5 pour purifier en lit fixe l'AMCA.

Le brevet US 2 863 917 décrit aussi la purification de l'AMCA en phase liquide par du charbon actif en poudre chargé de palladium dans un réacteur agité.

Le brevet DE 1 915 037 décrit la purification de l'AMCA par des catalyseurs de diamètre 5 mm en lit fixe à base de silice chargée de palladium et aussi de la poudre de silice de 50 à 150 microns chargée de palladium dans un réacteur agité.

Le brevet FR 2 027 078 décrit aussi la purification de l'AMCA en phase liquide dans un réacteur agité en présence de poudre de silice de 40 à 200 microns, chargée de palladium.

Le brevet US 3 304 325 concerne la purification de l'AMCA en phase vapeur sur du charbon actif seul.

Dans le brevet EP 453690, on décrit un catalyseur de deshalogénation d'acides carboxyliques alphahalogénés à base d'un métal précieux déposé sur un charbon se présentant sous forme de cylindres extrudés de diamètre égal à 2 mm et de longueur égale à 4 mm.

La demanderesse a trouvé un nouveau catalyseur utile pour la deshalogénation des acides carboxyliques alphahalogénés qui permet une réaction plus sélective qui diminue les sous-produits et améliore fortement la productivité.

La présente invention est donc un catalyseur constitué d'un support en charbon actif sous forme de particules cylindriques dont au moins 85% en poids de l'ensemble du catalyseur ont de 0,3 à 1,5 mm de diamètre et 0,3 à 5 mm de longueur les dites particules étant chargées d'un métal précieux du groupe 8 de la classification périodique.

Les métaux précieux du groupe VIII de la classification périodique des éléments sont le ruthénium, le rhodium, le palladium, l'osmium, l'iridium et le platine. On utilise avantageusement les trois premiers et de préférence le palladium. Ces métaux peuvent être utilisés seuls, en alliages ou en mélange entre eux.

Le métal précieux est déposé sur un charbon actif c'est-à-dire un charbon de grande surface à raison de 0,3 à 1% en poids du catalyseur, c'est-à-dire du charbon plus du métal et il est réparti en surface du charbon. On entend par charbon de grande surface un charbon d'environ 600 $m^2/g$ et pouvant aller jusqu'à 1300 $m^2/g$.

On peut aussi de façon plus simple traduire la taille des particules en nombre de particules de diamètre par $cm^3$, quand ces particules sont en vrac dans un récipient.

Avantageusement, le catalyseur de l'invention est constitué de particules cylindriques de taille comprise entre 0,7 et 1,2 mm de diamètre. Ce catalyseur peut être en tout ou partie dopé par du soufre ou des composés du soufre comme décrit dans EP 0 453 690. Les avantages de ces catalyseurs sont décrits plus bas dans le procédé.

La présente invention concerne aussi l'utilisation de ces catalyseurs, c'est-à-dire un procédé de deshalogénation d'acides carboxyliques alphahalogénés.

La présente invention est donc un procédé de deshalogénation d'acides carboxyliques alphahalogénés ou de leurs esters par l'hydrogène caractérisé en ce qu'on opère en présence du catalyseur qu'on vient de décrire.

L'invention est particulièrement utile pour les acides de formule :

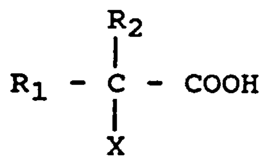

(I)

dans laquelle X est le chlore ou le brome, $R_1$ et $R_2$ sont identiques ou différents et représentent le chlore, le brome, H, un radical alkyle linéaire ou ramifié ayant de 1 à 12 carbones ou un radical cycloalkyle ayant de 3 à 12 carbones. L'invention s'applique aussi aux esters des acides de formule (I). Ce sont de préférence les esters aliphatiques ayant de 1 à 10 carbones, et de préférence 1 à 5 atomes de carbone.

Selon l'invention, on peut effectuer la déshalogénation d'un acide ou d'un mélange d'acides. Ces acides peuvent être aussi en mélange avec un solvant.

Selon les applications des acides deshalogénés, il est parfois nécessaire de séparer-en fin de déshalogénation le catalyseur des acides. Selon une forme avantageuse de l'invention, le catalyseur est disposé en lit fixe ou en lit fluide dans une capacité et les acides à déshalogéner et l'hydrogène sont introduits dans cette capacité. Il n'est donc pas nécessaire en fin de déshalogénation de séparer le catalyseur. De préférence, on utilise un cata en lit fixe et on opère en continu.

Il est avantageux d'opérer le procédé de l'invention avec les acides en phase liquide. Bien qu'on puisse opérer à toute température, il est avantageux d'être entre la température à laquelle les acides sont liquides et 200°C, et de préférence entre 100 et 180°C. Si nécessaire, on peut mettre le ou les acides dans un solvant pour pouvoir se placer dans cette zone de température préférée.

On peut opérer à la pression atmosphérique ou jusqu'à 5 bars. L'effet de la pression est d'augmenter la cinétique de réaction, comme les acides sont corrosifs et aussi le milieu réactionnel de l'invention, il n'est pas judicieux de dépasser une pression de l'ordre de 5 bars.

L'invention est particulièrement utile pour purifier des acides carboxyliques monoalphahalogénés $R_1CHXCOOH$ impurs, $R_1$ ayant la signification précédente. Ces acides sont préparés par halogénation de l'acide correspondant $R_1CH_2COOH$, on obtient un mélange de $R_1CHXCOOH$, de $R_1CX_2COOH$, d'acide $R_1CH_2COOH$ qui n'a pas été transformé et parfois des traces de $CX_3COOH$ dans le cas particulier de l'acide $CH_3COOH$.

On pourrait d'abord séparer $R_1CH_2COOH$ de ce mélange mais il est plus simple d'hydrogéner d'abord

$$R_1CX_2COOH + H_2 \rightarrow R_1CHXCOOH + HX$$

et séparer ensuite, puisque inévitablement une partie est rétrogradée en acide selon

$$R_1CHXCOOH + H_2 \rightarrow R_1CH_2COOH + HX$$

Il suffit ensuite de distiller le mélange de $R_1CHXCOOH$, de $R_1CH_2COOH$ et d'HX pour obtenir $R_1CHXCOOH$ relativement pur. Au cours de l'hydrogénation, il se forme des aldéhydes, par exemple, $R_1CH_2CHO$.

La rétrogradation est le rapport entre le nombre d'ions $X^-$ dans l'acide purifié, c'est-à-dire ceux provenant de HX au nombre théorique de X à enlever de $R_1CX_2COOH$ (et éventuellement $CX_3COOH$) pour le convertir en $R_1CHXCOOH$. Sauf exception de $CX_3COOH$, la rétrogradation minimum est de 1. Cette rétrogradation est le plus souvent entre 1,4 et 3,4.

Le catalyseur de l'invention permet de baisser la quantité d'aldéhydes et d'améliorer la rétrogradation, tout en utilisant la même quantité de palladium. La taille du catalyseur de l'invention permet d'opérer en lit fixe sans une perte de charge gênante.

La présente invention concerne aussi un procédé dans lequel on utilise le catalyseur de l'invention en série avec un catalyseur de plus grande taille.

Ce catalyseur de plus grande taille est un support en charbon actif sous forme de particules chargé de métal précieux du groupe 8. La grande taille peut être définie par des cylindres de diamètre supérieur à 2 mm et de longueur supérieure à 3 mm et par des sphères de diamètre supérieur à 3 mm. Il suffit que 85 à 95 % en poids d'un lot de

catalyseur ait ces dimensions pour qu'il soit "de plus grande taille" au sens de l'invention.

On peut utiliser un courant d'hydrogène à co-courant du courant d'acides ou à contre courant.

EXEMPLE 1 :

Dans un équipement constitué par deux réacteurs tubulaires en verre, de diamètre intérieur 26 mm et volume 300 $cm^3$, chauffé par double enveloppes et équipés d'un matériel permettant de procéder à l'alimentation à co ou contre courant, d'acide monochloracétique à purifier et d'hydrogène à des débits parfaitement réguliers, on place dans l'un des réacteur **A** un catalyseur constitué de grains de charbon actif imprégné à 0,8% de palladium, 17,25 grains au $cm^3$ et de surface spécifique voisine de 1200 $m^2/g$ et ayant une masse volumique de 0,433 $g/cm^3$.

Dans le réacteur **B** on place un catalyseur de même densité, surface spécifique, masse volumique et teneur en palladium, mais constitué de 408 grains au cm3, c'est-à-dire que les catalyseurs A et B contiennent la même quantité de palladium. On met ensuite le système en fonctionnement à contre courant, en recherchant pour l'acide dichloracétique, une teneur résiduelle inférieure à 0,2%. Pour cela, on agit sur la température. Les débits de liquide, gaz et hydrogène demeurent constants.

Acide monochloracétique à purifier (% poids) :

| | |
|---|---|
| Acide monochloracétique (AMCA) : | 80 |
| Acide dichloracétique (ADCA) : | 4 |
| Acide acétique : | 16 |

Le catalyseur **A** est de tailleDiamètre 3 mm
Longueur 10 mm
Le catalyseur **B** est de tailleDiamètre 1 mm
Longueur 3 mm

Le tableau I ci-après constitué de deux relevés de marche du système, montre que le catalyseur le plus fin permet de travailler en plus faible température et génère moins de sous produits.

TABLEAU I

| | Température 0°C | Vitesse spatiale Kg/h/m3 | ADCA résiduel % | Teneur MAAG % | Aldéhydes générés mg/Kg | Rétrogradation |
|---|---|---|---|---|---|---|
| 1A Réacteur A | 125 | 245 | 0,15 | 0,46 | 850 | 2,47 |
| 2A Réacteur A | 140 | 465 | 0,07 | 0,6 | 723 | 2,89 |
| 3A Réacteur A | 140 | 475 | 0,12 | 0,50 | 1170 | 2,54 |
| 1B Réacteur B | 110 | 250 | 0,10 | ≤0,09 | 490 | 2,3 |
| 2B Réacteur B | 115 | 494 | 0,09 | ≤0,05 | 413 | 1,83 |
| 3B Réacteur B | 120 | 823 | 0,17 | ≤0,05 | 550 | 2,1 |

**Retrogradation** = rapport entre les chlorures totaux et les chlorures provenant de la réduction ADCA en AMCA
**Les aldehydes** sont exprimés en acétaldehyde
Le catalyseur A n'est pas conforme à l'invention

Dans le tableau I :

- Les indications 1A, 1B indiquent les valeurs à des temps identiques.
- La température de la colonne est la température relevée par un thermocouple dans le lit du catalyseur.
- La vitesse spatiale est en kg d'acide brut par heure et volume de lit de catalyseur.
- La teneur en MAAG (monochloracétate d'acide glycolique) est en sortie de réacteur dans l'acide purifié.

EXEMPLE 2 :

Les deux réacteurs de l'exemple 1 contenant les mêmes catalyseurs sont disposés en série pour purifier de l'AMCA de même composition que dans l'exemple 1.

2.1. L'AMCA brut passe d'abord sur le catalyseur de petite taille (Résultats sur le tableau II). On a recherché la température de fonctionnement pour avoir une teneur finale en ADCA inférieure ou égale à 0,2%. Les mesures suivantes ont été faites entre la 225ème et la 346ème heure de marche.

TABLEAU II

| | Exemple 2.1 | Exemple 2.2 |
|---|---|---|
| Vitesse spatiale AMCA brut | 250 kg/h m3 | 250 kg/h m3 |
| Température catalyseur (sur les deux réacteurs) | 115°C | 115°C |
| ADCA en sortie du 1er réacteur | 0,25 à 0,33 | 1,1 à 1,2 |
| ADCA en sortie du 2ème réacteur | 0,10 à 0,13 | 0,07 à 0,11 |
| Acétaldéhyde généré mg/kg d'acide | 120 à 140 | 85 à 89 |

2.2. L'AMCA passe d'abord sur le catalyseur de grande taille.
On garde la température de 115°C.
On note le gain très important de l'aldéhyde par rapport à l'exemple 1.

## Revendications

1. Catalyseur constitué d'un support en charbon actif sous forme de particules cylindriques dont au moins 85% en poids de l'ensemble du catalyseur ont de 0,3 à 1,5 mm de diamètre et 0,3 à 5 mm de longueur les dites particules étant chargées d'un métal précieux du groupe 8 de la classification périodique.

2. Produit selon la revendication 1 caractérisé en ce que le métal précieux est du palladium.

3. Produit selon la revendication 1 ou 2 caractérisé en ce que le diamètre des particules est de 0,7 à 1,2 mm.

4. Procédé de déshalogénation d'acides carboxyliques alphahalogénés ou de leurs esters par l'hydrogène caractérisé en ce qu'on opère en présence d'un catalyseur selon les revendications 1 à 3.

5. Procédé selon la revendication 4 caractérisé en ce que les acides sont de formule
$R_1CHXCOOH$ dans laquelle X représente le chlore ou le brome, $R_1$ représente le chlore, le brome, H ou un radical alkyle linéaire ou ramifié ayant de 1 à 12 carbones ou un radical cycloalkyle ayant de 3 à 13 carbones.

6. Procédé selon la revendication 4 ou 5 caractérisé en ce que les acides sont en phase liquide.

7. Procédé selon l'une des revendications 4 à 6 caractérisé en ce qu'on utilise aussi en série un catalyseur de plus grande taille.

## Patentansprüche

1. Katalysator, bestehend aus einem Trägermaterial aus Aktivkohle in Form zylindrischer Teilchen, von denen mindestens 85 Gew.-% des gesamten Katalysators einen Durchmesser von 0,3 bis 1,5 mm und eine Länge von 0,3 bis 5 mm aufweisen, wobei diese Teilchen mit einem Edelmetall aus der Gruppe 8 des Periodensystems der Elemente beladen sind.

2. Produkt nach Anspruch 1, dadurch gekennzeichnet, daß das Edelmetall Palladium ist.

3. Produkt nach Anspruch 1 oder 2, dadurch gekennzeichnet, daß der Durchmesser der Teilchen 0,7 bis 1,2 mm

beträgt.

4. Verfahren zur Dehalogenierung von α-halogenierten Carbonsäuren oder ihren Estern mittels Wasserstoff, dadurch gekennzeichnet, daß man in Gegenwart eines Katalysators nach den Ansprüchen 1 bis 3 verfährt.

5. Verfahren nach Anspruch 4, dadurch gekennzeichnet, daß die Säuren die Formel $R_1CHXCOOH$ aufweisen, in der X Chlor oder Brom darstellt; $R_1$ Chlor, Brom, H oder einen linearen oder verzweigten Alkylrest mit 1 bis 12 Kohlenstoffatomen oder einen Cykloalkylrest mit 3 bis 13 Kohlenstoffatomen darstellt.

6. Verfahren nach Anspruch 4 oder 5, dadurch gekennzeichnet, daß die Säuren als flüssige Phasen vorliegen.

7. Verfahren nach einem der Ansprüche 4 bis 6, dadurch gekennzeichnet, daß man in Serie auch einen Katalysator mit größerer Dimension verwendet.

**Claims**

1. Catalyst composed of an active charcoal support in the form of cylindrical particles of which at least 85% by weight of the catalyst as a whole have a diameter from 0.3 to 1.5 mm and a length of 0.3 to 5 mm, the said particles being loaded with a precious metal from group 8 of the Periodic Table.

2. Product according to Claim 1, characterized in that the precious metal is palladium.

3. Product according to Claim 1 or 2, characterized in that the diameter of the particles is from 0.7 to 1.2 mm.

4. Process for the dehalogenation of alpha-halogenated carboxylic acids or their esters by hydrogen, characterized in that the dehalogenation is carried out in the presence of a catalyst according to Claims 1 to 3.

5. Process according to Claim 4, characterized in that the acids are of formula

$$R_1CHXCOOH$$

in which X represents chlorine or bromine, $R_1$ represents chlorine, bromine, H or a linear or branched alkyl radical having from 1 to 12 carbon atoms or a cycloalkyl radical having from 3 to 13 carbon atoms.

6. Process according to Claim 4 or 5, characterized in that the acids are in the liquid phase.

7. Process according to one of Claims 4 to 6, characterized in that there is also used, in series, a catalyst of larger size.